## Europäisches Patentamt
## European Patent Office
## Office européen des brevets

(19)

(11) Numéro de publication: **0 076 715**
**B1**

(12) **FASCICULE DE BREVET EUROPEEN**

(45) Date de publication du fascicule du brevet:
**27.12.84**

(21) Numéro de dépôt: **82401665.3**

(22) Date de dépôt: **14.09.82**

(51) Int. Cl.³: **C 07 C 125/073**, C 08 G 18/10,
C 08 G 18/80

(54) **Toluène diisocyanate brut modifié permettant d'obtenir des mousses de polyuréthannes à haute résilience améliorées.**

(30) Priorité: **25.09.81 FR 8118075**

(43) Date de publication de la demande:
**13.04.83 Bulletin 83/15**

(45) Mention de la délivrance du brevet:
**27.12.84 Bulletin 84/52**

(84) Etats contractants désignés:
**BE CH DE FR GB IT LI LU NL SE**

(56) Documents cités:
**FR - A - 1 423 572**
**FR - A - 2 029 628**
**FR - A - 2 352 011**

(73) Titulaire: **P C U K PRODUITS CHIMIQUES UGINE KUHLMANN, Service Propriété Industrielle Tour Manhattan, F-92087 Paris La Défense 2 Cédex 21 (FR)**

(72) Inventeur: **Bertrand, Jean Lucien Paul, 9 Avenue Plein Soleil, F-60260 Lamorlaye (FR)**
Inventeur: **Gaillard, Michel André, 25 Avenue du Vallois, F-60500 Chantilly (FR)**

(74) Mandataire: **Varniere-Grange, Monique et al, RHONE-POULENC RECHERCHES Service Brevets Chimie et Polymères Centre de Recherches de Saint-Fons 85, Avenue des Frères Perret B.P. 62, F-69192 St-Fons Cédex (FR)**

## Description

La présente invention concerne un toluènediisocyanate (TDI) brut modifié de façon telle que son utilisation dans la fabrication de mousses de polyuréthannes permet d'obtenir des mousses de haute résilience dont, en particulier, la fermeté est améliorée.

Le toluènediisocyanate brut modifié se présente sous forme de prépolymère auquel on a fait subir un vieillissement à chaud.

On sait que le TDI est préparé industriellement par phosgénation d'un mélange de toluènediamines ou d'un mélange de chlorhydrates de toluènediamines. Au cours de cette réaction, effectuée en présence de solvant, il se forme, en même temps que le TDI, des composés lourds par suite de réactions secondaires des TDI sur eux-mêmes ou sur les toluènediamines. Ces composés lourds sont constitués principalement de carbodiimides, urées, biurets, diisocyanates dimérisés et trimérisés, ainsi que de composés mixtes encore plus complexes. Ils ont cependant la caractéristique chimique commune de conserver une ou plusieurs fonctions −NCO libres réactives. Ce produit de phosgénation obtenu après élimination du solvant contient un nombre de fonctions −NCO/kg compris entre 8,5 et 11,2.

Divers avantages, tant économique que techniques, par exemple une meilleure résistance à la fatigue en flexion et une plus grande facilité de mise en œuvre, résultent de l'utilisation d'un produit de phosgénation brut, dont le solvant a seulement été éliminé, comme polyisocyanate dans la fabrication de mousses polyuréthannes. L'utilisation d'un TDI brut, produit non distillé de phosgénation ou, comme dans le cas du brevet français No 1470254, résultant du mélange de TDI purifié avec les composés lourds résiduaires de la phosgénation, présente cependant un inconvénient important du fait de son instabilité et de sa mauvaise conservation. Cet inconvénient donne lieu à la formation de composés secondaires qui confèrent aux mousses obtenues des propriétés physiques médiocres, en particulier en ce qui concerne la déformation résiduelle après compression.

Pour remédier à ces inconvénients, divers traitements des TDI bruts ont été proposés. En particulier, on peut citer le brevet français No 2253771, selon lequel le TDI brut est traité par un acide faible de façon à transformer les carbodiimides. On peut également citer le brevet français No 2073924, selon lequel on trimérise une partie diisocyanate en présence de dérivés de la guanidine.

Par ailleurs, on sait que la fabrication des mousses souples de polyuréthannes à haute résilience (souvent définies par un facteur d'indentation ou Sag Factor supérieur à 2,5) quoique bien connue, reste difficile. D'une part, dans la composition des formulations, il n'entre pas de stabilisants, tels que des tensio-actifs résultant de la condensation d'oxyde d'alcoylène sur des siloxanes, habituellement utilisés pour la fabrication de mousses souples de polyuréthannes, mais seulement à la rigueur des huiles siliconées peu actives. D'autre part, la mousse finie, surtout dans le sac de la coulée de bloc en continu, ne doit pas comporter une trop grande quantité de cellules fermées qui entraîne une mauvaise stabilité dimensionnelle du bloc.

Le TDI brut modifié selon la présente invention non seulement possède les avantages connus propres aux TDI bruts, mais encore permet de fabriquer facilement des mousses souples de haute résilience avec des valeurs d'indentation améliorées.

Le traitement du TDI brut consiste, dans une première étape, à faire réagir sur le TDI brut un polyol en quantité telle que l'abaissement de la concentration en isocyanate du TDI brut soit au plus de 2,0 fonctions −NCO/kg de TDI brut, et de préférence compris entre 0,3 et 0,8, puis, dans une seconde étape, à faire vieillir à chaud le prépolymère pendant une durée telle que l'abaissement de la concentration en isocyanate du prépolymère soit au plus de 0,4 fonction −NCO/kg de prépolymère.

Le TDI brut initial à traiter possède, de préférence, une concentration en isocyanate pouvant varier entre 9,0 et 11,0 fonctions −NCO/kg. Il provient directement de la réaction de phosgénation après élimination du solvant et éventuellement après extraction partielle de TDI pur. On considère également comme TDI brut tout mé-lange de TDI pur et de produits lourds résiduels de la réaction de phosgénation permettant de réaliser de façon synthétique un produit possédant un nombre de fonctions −NCO libres/kg en quantité convenable pour réaliser le TDI brut modifié selon l'invention.

Les polyols utilisables dans la première étape ont de préférence un poids moléculaire équivalent (PME) d'environ 30 à 1000 et une fonctionnalité de 2 à 8 groupes hydroxyliques. On entend par PME le quotient du poids moléculaire du polyol par sa fonctionnalité. Ces polyols sont maintenant bien connus et correspondent à ceux couramment utilisés dans la fabrication des polyuréthannes. A titre purement indicatif, on peut citer le glycérol, le triméthylolpropane, l'hexanetriol, le pentaérythri-tol, le sorbitol, le saccharose, les hexols, ainsi que les dérivés d'oxyalcoylénation de ces polyols.

La réaction du TDI brut sur le polyol est bien connue en elle-même, s'agissant d'une prépoly-mérisation. Cette réaction s'effectue de préférence sous atmosphère sèche dans un réacteur muni d'un agitateur et d'un moyen de régulation de la température. Habituellement, au TDI brut préchauffé à une température inférieure à 70° C, on ajoute le polyol, puis on mélange sous relativement lente agitation, tout en maintenant le milieu réactionnel à une température généralement inférieure à 100° C pendant le temps suffisant pour obtenir le titre en −NCO visé. Le produit obtenu est, dans la seconde étape, stocké, sous atmosphère de préférence inerte, à une température comprise habituellement entre 50 et 110° C pendant le temps nécessaire pour amener la concentration en isocyanate du produit de la première étape au titre choisi pour le TDI brut modifié final. Le temps nécessaire pour abaisser la concentration en isocyanate du prépolymère est fonction de la température et peut varier d'environ quelques heures à un mois.

Le TDI brut modifié ainsi obtenu est utilisable dans les conditions de tout autre TDI pour la fabrication de mousses souples à haute résilience.

Les exemples suivants servent à illustrer l'invention.

*Exemple 1 :*

Dans un réacteur type Grignard de 60 l, on charge 50 kg de TDI brut à 9,92 fonctions −NCO/kg obtenu par mélange de TDI riche en produits lourds et de TDI pur. La viscosité mesurée à 25°C au Brookfield, modèle LVT, vitesse 60 tr/min, aiguille N° 1, est de 15 cPo. On chauffe sous azote cet isocyanate à 42°C. On ajoute 1,690 kg de polyol triol de poids moléculaire d'environ 330 et d'indice d'hydroxyde 505, obtenu par oxypropylation de la glycérine. On maintient sous agitation cette température pendant 15 min, puis on soutire le produit dans un fût dont la surface est maintenue sous azote. Après 24 h, on détermine par la méthode AFNOR T 52 133 la concentration en isocyanate de ce TDI brut qui est de 9,28 fonctions −NCO/kg, la viscosité étant de 40 cPo à 25°C. Le fût est placé pendant 30 d dans une étuve réglée à 60°C. Après ce temps, le titre final de ce TDI brut modifié est tombé à 9,03 fonctions −NCO/kg et la viscosité est de 51 cPo à 25°C, les conditions de mesure au viscosimètre Brookfield étant: aiguille N° 1 - vitesse 60 tr/min.

On prépare à 22°C ±1 les différents mélanges moussants M par mélange des constituants suivants:

*(Tableau en tête de la colonne suivante)*

Le polyol A est un polyéther triol d'indice d'hydroxyle égal à 35, oxyéthylé et oxypropylé avec un rapport oxyde de propylène/oxyde d'éthylène égal à 86/14 et contenant une proportion de groupement hydroxyle primaire de 80%.

Le polyol B est un polyéther triol d'indice d'hydroxyle égal à 42, oxyéthylé et oxypropylé avec un rapport oxyde de propylène/oxyde d'éthylène égal à 25/75 et contenant une proportion de groupement hydroxyle primaire de 45%.

Le polyol C est un polyéther triol d'indice d'hydroxyle égal à 42, oxypropylé et oxyéthylé avec un rapport oxyde de propylène/oxyde d'éthylène égal à 86/14, mais ne contenant pas de groupement hydroxyle primaire.

Le Niax A 1 est un catalyseur pour mousses de polyuréthannes à base de bis-(2-diméthylamino-éthyl)éther.

Le Tegostab 4380 est un produit à base d'huile de silicone.

Le Tegostab 2370 est un tensio-actif, produit de condensation d'oxyde d'alcoylène sur des siloxanes.

Les mélanges moussants M étant homogènes, on ajoute les doses respectives d'octoate stanneux. Après nouvelle homogénéisation, on intro-

| Constituants (parties en poids) | | Essais | | | |
|---|---|---|---|---|---|
| | | 1 | 2 | 3 | 4 |
| Mélanges M | Polyol A | 0 | 93 | 90 | 90 |
| | Polyol B | 0 | 0 | 2 | 2 |
| | Polyol C | 100 | 0 | 0 | 0 |
| | Eau | 3,4 | 3,2 | 3,5 | 3,5 |
| | Diéthanolamine | 0 | 3,5 | 3,5 | 3,5 |
| | Diméthyl-éthanolamine | 0,3 | 0,3 | 0,4 | 0,4 |
| | Catalyseur Niax A 1 | 0 | 0,1 | 0 | 0 |
| | Tegostab 4380 | 0 | 0,3 | 0,2 | 0,2 |
| | Tegostab 2370 | 0,8 | 0 | 0 | 0 |
| Catalyseur: octoate stanneux | | 0,22 | 0,03 | 0,08 | 0,15 |
| TDI pur (isomères 2,4, 2,6 = 80/20) | | 42,0 | 0 | 0 | 0 |
| TDI brut (initial à 9,92 fonctions −NCO/kg) | | 0 | 51,6 | 0 | 0 |
| TDI brut après la 1re étape (9,28 fonctions −NCO/kg) | | 0 | 0 | 59,0 | 0 |
| TDI brut modifié final | | 0 | 0 | 0 | 60,7 |

duit la quantité de chaque TDI correspondant à chacun des essais. Après agitation pendant 8 s, on coule dans des moules ouverts de 45 × 45 cm de section et de 40 cm de hauteur.

Les mousses obtenues présentent les caractéristiques suivantes:

| | | Essais | | |
|---|---|---|---|---|
| | 1 | 2 | 3 | 4 |
| Masse volumique (kg/m³) | 30 | 30,2 | 31,0 | 30,5 |
| Force (N) appliquée pour obtenir une indentation de 50% | 230 | 85 | 115 | 225 |
| Facteur d'indentation | 2,1 | 2,7 | 3,0 | 2,6 |

La masse volumique est déterminée suivant la norme AFNOR T 56 107 et les valeurs d'indentation suivant la norme AFNOR T 56 111. Cependant, pour mieux caractériser la fermeté des mousses, il a été choisi une indentation de 50%, au lieu de 40% habituellement, déterminée sur des éprouvettes d'une épaisseur de 10 cm au lieu de 5 cm.

Les quatre essais conduisent à des mousses dont les densités sont sensiblement les mêmes. L'essai 1 est une mousse de qualité standard. Les essais 2 et 3 donnent des mousses de qualité haute élasticité, mais difficile à obtenir et de fermeté faible. L'essai 4, avec le TDI brut modifié selon l'invention, donne une mousse de qualité haute élasticité facile à obtenir et avec une fermeté améliorée.

*Exemple 2:*

On procède selon l'exemple 1 pour la fabrication du produit de la première étape. 24 h après soutirage, le produit titre 9,27 fonctions −NCO/kg et sa viscosité est de 39 cPo à 25° C. Ce produit est alors placé pendant 19 d sous azote dans une étuve réglée à 80° C; après ce temps, le titre final de TDI brut modifié est tombé à 9,0 fonctions −NCO/kg et la viscosité est de 60 cPo à 25° C.

Avec ce TDI brut modifié, on reproduit l'essai 4 de l'exemple 1; on obtient facilement une mousse de qualité haute élasticité dont les caractéristiques sont les suivantes:
— Masse volumique: 32 kg/m³
— Force appliquée pour obtenir une indentation de 50%: 210 N
— Facteur d'indentation: 2,7.

*Exemple 3:*

On procède selon l'exemple 1 pour la fabrication du produit de la première étape. 24 h après soutirage, le produit titre 9,26 fonctions −NCO/kg et sa viscosité est de 36 cPo à 25° C. Ce produit est alors placé pendant 8 d sous azote dans une étuve réglée à 100° C; après ce temps, le titre final du TDI brut modifié est tombé à 8,98 fonctions −NCO/kg et la viscosité est de 58 cPo à 25° C.

Avec ce TDI brut modifié, on reproduit l'essai 4 de l'exemple 1; on obtient facilement une mousse de qualité haute élasticité dont les caractéristiques sont les suivantes:
— Masse volumique: 31 kg/m³
— Force appliquée pour obtenir une indentation de 50%: 230 N
— Facteur d'indentation: 2,7.

*Exemple 4:*

On charge, dans un réacteur type Grignard de 60 l, 20 kg de TDI brut à 10,0 fonctions −NCO/kg. La viscosité mesurée à 25° C au Brookfield modèle LVT, vitesse 60 tr/min - aiguille No 1, est de 17 cPo. On chauffe sous azote cet isocyanate à 40° C. On ajoute 0,471 kg de polyol du type sorbitol oxypropylé jusqu'à un poids moléculaire d'environ 700 et d'indice d'hydroxyle égal à 498. On maintient sous agitation cette température pendant 15 min, puis on soutire tout le produit dans un fût dont la surface est maintenue sous azote. Après 24 h, on détermine par la méthode AFNOR T 52 133 la concentration en isocyanate de ce TDI brut, et on trouve 9,52 fonctions −NCO/kg et une viscosité de 30 cPo à 25° C. Le fût est alors placé pendant 11 d dans une étuve réglée à 100° C. Après ce temps, le titre final de ce TDI brut modifié est tombé à 9,22 fonctions −NCO/kg et la viscosité est de 46 cPo - conditions de mesure au viscosimètre Brookfield: aiguille No 1, vitesse 60 tr/min.

Avec ce TDI brut modifié, on reproduit l'essai 4 de l'exemple 1; on obtient facilement une mousse de qualité haute élasticité dont les caractéristiques sont les suivantes:
— Masse volumique: 32,3 kg/m³
— Force appliquée pour obtenir une indentation de 50%: 135 N

— Facteur d'indentation: 2,8.

*Exemple 5:*

On charge, dans un réacteur type Grignard de 60 l, 20 kg de TDI brut à 10,0 fonctions −NCO/kg obtenu par mélange de toluènediisocyanate riche en produits lourds et de toluènediisocyanate pur. La viscosité mesurée au Brookfield modèle LVT, vitesse 60 tr/min - aiguille No 1, est de 17 cPo. On chauffe cet isocyanate sous azote à 40° C. On ajoute 1,346 kg de polyol tétrol obtenu par polyaddition de 85% en poids d'oxyde de propylène, puis de 15% en poids d'oxyde d'éthylène sur de l'éthylènediamine jusqu'à un poids moléculaire d'environ 3600 et d'indice d'hydroxyle égal à 62. On maintient sous agitation cette température 15 min, puis on soutire tout le produit dans un fût maintenu sous azote. Après 24 h, on détermine par la méthode AFNOR T 52 133 la concentration en isocyanate de ce TDI brut et on trouve 9,26 fonctions −NCO/kg et une viscosité de 32 cPo à 25° C. Le fût est alors placé pendant 6 d dans une étuve réglée à 100° C. Après ce temps, le titre final de ce TDI brut modifié est tombé à 8,93 fonctions −NCO/kg et la viscosité est de 46 cPo.

Avec ce TDI brut modifié, on reproduit l'essai 4 de l'exemple 1; on obtient facilement une mousse de qualité haute élasticité dont les caractéristiques sont les suivantes:
— Masse volumique: 28,9 kg/m³
— Force appliquée pour obtenir une indentation de 50%: 162 N
— Facteur d'indentation: 2,9.

*Exemple 6:*

On charge, dans un réacteur type Grignard de 60 l, 20 kg de TDI brut à 10,95 fonctions −NCO/kg obtenu par mélange de toluènediisocyanate riche en produits lourds et de toluène diisocyanate pur. La viscosité mesurée au Brookfield, modèle LVT, vitesse 60 tr/min - aiguille No 1, est de 9 cPo. On chauffe cet isocyanate à 40° C sous azote. On ajoute 0,727 kg de polyol triol obtenu par oxypropylation de la glycérine jusqu'à un poids moléculaire d'environ 330 et d'indice d'hydroxyle égal à 505. On maintient sous agitation cette température 20 min, puis on soutire tout le produit dans un fût dont la surface est maintenue sous azote. Après 24 h, on détermine par la méthode AFNOR T 52 133 la concentration en isocyanate de ce TDI brut et on trouve 10,27 fonctions −NCO/kg et une viscosité de 11 cPo à 25° C. Le fût est alors placé pendant 3 d dans une étuve réglée à 100° C. Après ce temps, le titre final de ce TDI brut modifié est tombé à 9,98 fonctions −NCO/kg et la viscosité est de 13 cPo.

Avec ce TDI brut modifié, on reproduit l'essai 4 de l'exemple 1, sauf que l'on ajoute 54,9 parties de TDI brut modifié au lieu de 60,7 parties de l'essai 4; on obtient facilement une mousse de qualité haute élasticité dont les caractéristiques sont les suivantes:
— Masse volumique: 29,1 kg/m³
— Force appliquée pour obtenir une indentation de 50%: 130 N

— Facteur d'indentation: 2,7.

## Revendications

1. Toluènediisocyanate brut modifié, caractérisé en ce qu'il est obtenu par réaction, dans une première étape, d'un toluènediisocyanate brut avec un polyol en quantité telle que l'abaissement de la concentration en isocyanate de toluènediisocyanate brut soit au plus de 2,0 fonctions −NCO/kg de toluènediisocyanate brut, et en ce que, dans une seconde étape, le produit de réaction précédent est vieilli à chaud pendant une durée telle que l'abaissement de la concentration en isocyanate de ce produit de réaction soit au plus de 0,4 fonction −NCO/kg de produit.

2. Toluènediisocyanate modifié selon la revendication 1, caractérisé en ce que le toluènediisocyanate brut de la première étape possède une concentration en isocyanate de 9,0 à 11,0 fonctions −NCO/kg.

3. Toluènediisocyanate modifié selon l'une des revendications 1 ou 2, caractérisé en ce que le toluènediisocyanate de la première étape est un mélange de toluènediisocyanate pur et de produits lourds résiduels de la réaction de phosgénation.

4. Toluènediisocyanate modifié selon l'une des revendications 1 à 3, caractérisé en ce que le polyol de la première étape possède une fonctionnalité de 2 à 8 groupes hydroxyliques.

5. Toluènediisocyanate modifié selon l'une des revendications 1 à 4, caractérisé en ce que le polyol de la première étape possède un poids moléculaire équivalent d'environ 30 à 1000.

6. Toluènediisocyanate modifié selon l'une des revendications 1 à 5, caractérisé en ce que le produit de réaction de la première étape est vieilli dans une seconde étape à une température comprise entre 50 et 110° C.

## Patentansprüche

1. Modifiziertes rohes Toluendiisocyanat, dadurch gekennzeichnet, dass es erhalten worden ist durch Umsetzung in einer ersten Stufe eines rohen Toluendiisocyanats mit einem Polyol in solcher Menge, dass die Abnahme der Isocyanatkonzentration des rohen Toluendiisocyanats höchstens 2,0 NCO-Gruppen/kg rohes Toluendiisocyanat beträgt, und dadurch, dass in einer zweiten Stufe das in der ersten Stufe erhaltene Reaktionsprodukt in der Wärme während einer solchen Zeitspanne gealtert wird, dass die Abnahme der Isocyanatkonzentration dieses Reaktionsproduktes höchstens 0,4 NCO-Gruppen/kg Produkt beträgt.

2. Modifiziertes Toluendiisocyanat nach Anspruch 1, dadurch gekennzeichnet, dass das rohe Toluendiisocyanat der ersten Stufe eine Isocyanatkonzentration von 9,0 bis 11,0 NCO-Gruppen/kg aufweist.

3. Modifiziertes Toluendiisocyanat nach einem der Ansprüche 1 oder 2, dadurch gekennzeichnet, dass das rohe Toluendiisocyanat der ersten Stufe ein Gemisch aus reinem Toluendiisocyanat und schweren Produkten ist, die bei der Phosgenierungsreaktion als Rückstand anfallen.

4. Modifiziertes Toluendiisocyanat nach einem der Ansprüche 1 bis 3, dadurch gekennzeichnet, dass das Polyol der ersten Stufe 2 bis 8 Hydroxylgruppen aufweist.

5. Modifiziertes Toluendiisocyanat nach einem der Ansprüche 1 bis 4, dadurch gekennzeichnet, dass das Polyol der ersten Stufe ein Molekularäquivalentgewicht von etwa 30 bis 1000 aufweist.

6. Modifiziertes Toluendiisocyanat nach einem der Ansprüche 1 bis 5, dadurch gekennzeichnet, dass das Reaktionsprodukt der ersten Stufe in einer zweiten Stufe bei einer Temperatur von 50 bis 110° C gealtert wird.

## Claims

1. Modified crude toluene diisocyanate characterised in that it is obtained by the reaction, in a first stage, of a crude toluene diisocyanate with a polyol in a quantity such that the lowering in the isocyanate concentration of the crude toluene diisocyanate is at most 2.0 NCO functions/kg of crude toluene diisocyanate and that, in a second stage, the preceding reaction product is aged hot for a time such that the lowering in the isocyanate concentration of this reaction product is at most 0.4 NCO function/kg of product.

2. Modified toluene diisocyanate according to Claim 1, characterised in that the crude toluene diisocyanate from the first stage has an isocyanate concentration of 9.0 to 11.0 NCO functions/kg.

3. Modified toluene diisocyanate according to one of Claims 1 or 2, characterised in that the crude toluene diisocyanate from the first stage is a mixture of pure toluene diisocyanate and residual heavy products from the phosgenation reaction.

4. Modified toluene diisocyanate according to one of Claims 1 to 3, characterised in that the polyol from the first stage has a functionality of 2 to 8 hydroxyl groups.

5. Modified toluene diisocyanate according to one of Claims 1 to 4, characterised in that the polyol from the first stage has a molecular weight equivalent to approximately 30 to 1000.

6. Modified toluene diisocyanate according to one of Claims 1 to 5, characterised in that the reaction product from the first stage is aged in a second stage at a temperature of between 50 and 110° C.